# EUROPEAN PATENT APPLICATION

(11) **EP 4 382 616 A1**
(43) Date of publication of application: **12.06.2024**
(21) Application number: 21956297.2
(22) Date of filing: 07.09.2021
(51) Int. Cl.: C12Q 1/6869

(54) **METHOD FOR ANALYZING SEQUENCE OF TARGET POLYNUCLEOTIDE**

(71) Applicant: MGI Tech Co., Ltd., Shenzhen, Guangdong 518083 (CN)
(72) Inventor: GONG, Meihua, SHENZHEN, Guangdong 518083 (CN); ZHANG, Mingxuan, SHENZHEN, Guangdong 518083 (CN); ZHOU, Shuang, SHENZHEN, Guangdong 518083 (CN); LONG, Xiaojuan, SHENZHEN, Guangdong 518083 (CN); XU, Chongjun, SHENZHEN, Guangdong 518083 (CN); JIANG, Hui, SHENZHEN, Guangdong 518083 (CN); LIU, Jian, SHENZHEN, Guangdong 518083 (CN)
(74) Representative: Inspicos P/S
(86) International application number: PCT/CN2021/116924
(87) International publication number: WO 2023/035108

(57) **Abstract**

The present invention relates to a method for analyzing a sequence of a target polynucleotide. Full polymerization is achieved by means of multiple times of polymerization of a nucleotide mixture and polymerase, and a marker is detected while the polymerization reaction is performed. Furthermore, the present invention relates to a kit, capable of being used for analyzing or sequencing a polynucleotide.

## Description

### Technical Field

The present invention relates to a method for analyzing a target polynucleotide sequence, comprising polymerizing a nucleotide mixture and a polymerase multiple times to achieve efficient and sufficient polymerization, and detecting a label while performing the polymerization reaction. Furthermore, the present invention also relates to a kit, which can be used to analyze or sequence a polynucleotide.

### Background Art

High-throughput sequencing, of which sequencing by synthesis (SBS) is the commercial mainstream, mainly uses DNA polymerase and nucleotides with reversible termination and fluorescent labels to identify DNA sequences. It can sequence hundreds of thousands to millions of DNA molecules in parallel at a time, and has advantages such as high throughput, fast detection speed, flexibility and versatility, and low cost.

Currently, the specific process of sequencing by synthesis comprises employing amplification or rolling circle replication to generate a large number of DNA templates, then anchoring specific sequencing primers, and adding DNA polymerases and fluorescently labeled nucleotides to the reaction system at the same time; or adding DNA polymerases and a mixture of fluorescently labeled nucleotides and non-fluorescently modified nucleotides to the reaction system at the same time. The 3'-OH of these dNTPs is protected, so only one dNTP can be added at a time. Each time a dNTP is added, the replication reaction of the DNA chain will stop, then the fluorescence signal will be excited and collected, and then a chemical reagent will be added to quench the fluorescence signal and the dNTP 3'-OH protecting group will be removed so that the next round sequencing reaction can be carried out.

However, this sequencing technology requires particularly high synthesis efficiency. Incomplete synthesis among multiple copies will cause signal confusion, thus affecting sequencing accuracy and sequencing read length. This is also one of the key technical issues currently faced by second-generation sequencing. As people demand shorter sequencing time and greater throughput, the reaction efficiency of polymerases is limited, especially the polymerization ability of nucleotides with fluorescent groups is limited, and these will cause the polymerization efficiency to affect the quality of sequencing. Once the polymerization efficiency cannot keep up with the sequencing speed, incomplete polymerization occurs in multiple copies, which will lead to signal confusion during sequencing, thus limiting the read length and accuracy of sequencing.

Therefore, there is a need to provide a method for sequencing polynucleotides to achieve a more efficient reaction and sequencing effect without increasing the overall reaction time, so as to improve the read length and accuracy of sequencing.

### Contents of the present invention

The present invention provides a solution for polymerizing nucleotides while collecting signals, thereby improving the polymerization efficiency and achieving a more efficient polymerization efficiency without changing the overall reaction time.

Therefore, in a first aspect, the present application provides a method for analyzing a target polynucleotide sequence, comprising:
(a) providing a target polynucleotide;
(b) contacting the target polynucleotide with a primer under a condition that allows hybridization or annealing, thereby forming a partial duplex comprising the target polynucleotide and the primer used as a growing chain;
(c) contacting the partial duplex with a polymerase and a first nucleotide mixture under a condition that allows the polymerase to perform a nucleotide polymerization reaction, thereby extending the growing chain, wherein the first nucleotide mixture comprises at least one kind of nucleotide labeled with a label,
   wherein each nucleotide in the first nucleotide mixture comprises on its ribose or deoxyribose moiety a protecting group capable of blocking nucleic acid chain extension (e.g., a protecting group attached via a 2'- or 3'-oxygen atom);
(d) contacting the product of the previous step with a polymerase and a second nucleotide mixture under a condition that allows the polymerase to perform a nucleotide polymerization reaction, thereby extending the growing chain, wherein the second nucleotide mixture comprises at least one (e.g., one, two, three or four) unlabeled nucleotide or irreversible blocking nucleotide, or a combination of the unlabeled nucleotide and irreversible blocking nucleotide;
   wherein each unlabeled nucleotide in the second nucleotide mixture comprises on its ribose or deoxyribose moiety a protecting group capable of blocking nucleic acid chain extension (e.g., a protecting group attached via a 2'- or 3'-oxygen atom);
   and, providing a photographic polymerization solution and contacting it with the second nucleotide mixture, and detecting the presence of a label in the product of step (c) through the photographic polymerization reaction;
(e) removing the protective group and label contained in the product of the previous step;
(f) optionally repeating steps (c) to (e) one or more times;
thereby, obtaining a sequence information of the target polynucleotide.

In certain embodiments, the photographic polymerization solution comprises the following reagents: a nucleic acid polymerase, a buffer reagent, a surfactant, and a reagent for reducing nucleic acid damage under laser photography.

In certain embodiments, the photographic polymerization solution comprises the following reagents: a nucleic acid polymerase, a buffer reagent, a reagent for nucleic acid hybridization (e.g., sodium chloride), a Mg²⁺-containing salt, a surfactant, and a reagent for reducing nucleic acid damage under laser photography.

In certain embodiments, in step (c), the extension is an extension using the target polynucleotide as a template. In certain embodiments, the extension is an extension of one nucleotide.

In certain embodiments, the first nucleotide mixture comprises a first nucleotide labeled with a first label, a second nucleotide labeled with a second label, a third nucleotide labeled with a third label, and a fourth nucleotide labeled with a fourth label or an unlabeled fourth nucleotide, or comprises a first nucleotide labeled with a first label, a second nucleotide labeled with a second label, a third nucleotide co-labeled with the first label and the second label, and an unlabeled fourth nucleotide.

In certain embodiments, in step (d), the second nucleotide mixture comprises an unlabeled first nucleotide, an unlabeled second nucleotide, an unlabeled third nucleotide.

In certain embodiments, the second nucleotide mixture further comprises an unlabeled fourth nucleotide.

In certain embodiments, the second nucleotide mixture comprises at least one kind of irreversible blocking nucleotide.

In certain embodiments, in step (d), the second nucleotide mixture comprises an unlabeled first nucleotide, an unlabeled second nucleotide, an unlabeled third nucleotide, and a first irreversible blocking nucleotide, a second irreversible blocking nucleotide, a third irreversible blocking nucleotide, and a fourth irreversible blocking nucleotide.

In certain embodiments, the second nucleotide mixture further comprises an unlabeled fourth nucleotide.

In certain embodiments, the first label, the second label, the third label and the fourth label are each independently the same or different.

In certain embodiments, the first label, the second label, the third label and the fourth label are different.

In certain embodiments, the first label, the second label, the third label and the fourth label are luminescent labels (e.g., fluorescent labels).

In certain embodiments, the first label, the second label, the third label and the fourth label are each independently selected from the group consisting of coumarin, AlexaFluor, Bodipy, fluorescein, tetramethylrhodamine, phenoxazine, acridine, Cy5, Cy3, AF532, Texas Red and derivatives thereof.

In certain embodiments, the target polynucleotide comprises or is DNA, RNA, or any combination thereof. In certain embodiments, the extension product of the nucleic acid molecule is DNA.

In certain embodiments, the target polynucleotide is obtained from a sample derived from eukaryote (e.g., animal, plant, fungus), prokaryote (e.g., bacterium, actinomycete), virus, phage, or any combination thereof.

In certain embodiments, the first nucleotide, the second nucleotide, the third nucleotide and the fourth nucleotide are each independently selected from the group consisting of A, T, C, G, and U.

In certain embodiments, the first nucleotide, the second nucleotide, the third nucleotide and the fourth nucleotide are each different.

In certain embodiments, the first nucleotide, the second nucleotide, the third nucleotide and the fourth nucleotide are A, T, C, G, respectively.

In certain embodiments, the first irreversible blocking nucleotide, the second irreversible blocking nucleotide, the third irreversible blocking nucleotide and the fourth irreversible blocking nucleotide are each independently selected from A, T, C, G, U.

In certain embodiments, the first irreversible blocking nucleotide, the second irreversible blocking nucleotide, the third irreversible blocking nucleotide and the fourth irreversible blocking nucleotide are different.

In certain embodiments, the first irreversible blocking nucleotide, the second irreversible blocking nucleotide, the third irreversible blocking nucleotide and the fourth irreversible blocking nucleotide are A, T, C, G, respectively.

In certain embodiments, the irreversible blocking nucleotide is a dideoxynucleotide.

In certain embodiments, when the first nucleotide mixture comprises a first nucleotide labeled with a first label, a second nucleotide labeled with a second label, a third nucleotide labeled with a third label nucleotide and a fourth nucleotide labeled with a fourth label; the second nucleotide mixture comprises an unlabeled first nucleotide, an unlabeled second nucleotide, an unlabeled third nucleotide, an unlabeled fourth nucleotide; or,
when the first nucleotide mixture comprise a first nucleotide labeled with a first label, a second nucleotide labeled with a second label, a third nucleotide labeled with a third label, and an unlabeled fourth nucleotide, or comprises a first nucleotide labeled with a first label, a second nucleotide labeled with a second label, a third nucleotide co-labeled with the first label and the second label and an unlabeled fourth nucleotide, the second nucleotide mixture comprises an unlabeled first nucleotide, an unlabeled second nucleotide, and an unlabeled third nucleoside.

In some embodiments, taking photo to detect a light signal is performed while the polymerization reaction in step (d) is performed.

In certain embodiments, the buffer reagent is selected from the group consisting of Trizma base, tris(hydroxymethyl)methylglycine (TRICINE), *N*,*N*-dihydroxyethylglycine (BICINE), tris(hydroxymethyl)methylaminopropane sulfonic acid (TAPS), or any combination thereof.

In certain embodiments, the reagent for nucleic acid hybridization is selected from the group consisting of sodium chloride, potassium chloride, potassium acetate, or any combination thereof.

In certain embodiments, the Mg²⁺-containing salt is selected from the group consisting of magnesium sulfate, magnesium chloride, magnesium nitrate, magnesium chromate, or any combination thereof.

In certain embodiments, the surfactant is selected from the group consisting of 10% Tween 20, Tween 80, laureth (brij-35), polyethylene glycol octylphenyl ether (Trinton X-100), ethylphenyl polyethylene glycol (NP-40), sodium dodecyl sulfate (SDS), cetyltrimethylammonium bromide (CTAB), cetyltrimethylammonium chloride (CTAC), or any combination thereof.

In certain embodiments, the reagent for reducing nucleic acid damage under laser photography is selected from the group consisting of sodium ascorbate, dithiothreitol (DTT), 6-hydroxy-2,5,7,8-tetramethylchromane-2-carboxylic acid (Trolox), ascorbic acid derivative (e.g., sodium ascorbyl phosphate, magnesium ascorbyl phosphate, ascorbyl glucoside, ascorbyl palmitate, tetraethyldecanol ascorbyl, ascorbyl methylsilanol pectin acid ester, 3-O-ethyl ascorbic acid), reduced glutathione (GSH), *N*,*N'*-dimethylthiourea (DMTU), ammonium pyrrolidine dithiocarbamate (APDTC), spermidine, spermidine trihydrochloride, uric acid, sodium pyruvate, L-cysteine, β-mercaptoethylamine, cystamine, or any combination thereof.

In certain embodiments, the reagent for reducing nucleic acid damage under laser photography is selected from the group consisting of sodium ascorbate or reduced glutathione.

In one embodiment, the reagent for reducing nucleic acid damage under laser photography is selected from the group consisting of reduced glutathione (GSH) and dithiothreitol (DTT) and any combination thereof.

In one embodiment, the reagent for reducing nucleic acid damage under laser photography is reduced glutathione (GSH).

In a second aspect, the present application provides a kit, which comprises:
(a) a first nucleotide mixture, in which the first nucleotide mixture comprises at least one kind of nucleotide labeled with a label,
   wherein each nucleotide in the first nucleotide mixture comprises on its ribose or deoxyribose moiety a protecting group capable of blocking nucleic acid chain extension (e.g., a protecting group attached via a 2'- or 3'-oxygen atom);
(b) a second nucleotide mixture, in which the second nucleotide mixture comprises at least one (e.g., one, two, three, or four) unlabeled nucleotide or irreversible blocking nucleotide, or a combination of the unlabeled nucleotides and irreversible blocking nucleotides;
   wherein each unlabeled nucleotide in the second nucleotide mixture comprises on its ribose or deoxyribose moiety a protecting group capable of blocking nucleic acid chain extension (e.g., a protecting group attached via a 2'- or 3'-oxygen atom);
(c) a photographic polymerization solution.

In certain embodiments, the photographic polymerization solution comprises the following reagents: a nucleic acid polymerase, a buffer reagent, a surfactant, and a reagent for reducing nucleic acid damage under laser photography.

In certain embodiments, the photographic polymerization solution comprises the following reagents: a nucleic acid polymerase, a buffer reagent, a reagent for nucleic acid hybridization (e.g., sodium chloride), a Mg²⁺-containing salt, a surfactant, and a reagent for reducing nucleic acid damage under laser photography.

In certain embodiments, the kit further comprises one or more selected from the group consisting of: a primer complementary to all or part of the polynucleotide, a nucleic acid amplification buffer, a working buffer for enzyme (e.g., a nucleic acid polymerase), water, or any combination thereof.

In certain embodiments, the kit further comprises one or more selected from the group consisting of: a sequencing slide, a reagent for removing protecting group and label on nucleotide.

In certain embodiments, the kit is used for analyzing a polynucleotide.

In certain embodiments, the kit is used for sequencing a polynucleotide.

In certain embodiments, the first nucleotide mixture comprises a first nucleotide labeled with a first label, a second nucleotide labeled with a second label, a third nucleotide labeled with a third label and a fourth nucleotide labeled with a fourth label or an unlabeled fourth nucleotide, or comprises a first nucleotide labeled with a first label, a second nucleotide labeled with a second label, a third nucleotide co-labeled with the first label and the second label, and an unlabeled fourth nucleotide.

In certain embodiments, when the first nucleotide mixture comprises a first nucleotide labeled with a first label, a second nucleotide labeled with a second label, a third nucleotide labeled with a third label nucleotide and a fourth nucleotide labeled with a fourth label, the second nucleotide mixture comprises an unlabeled first nucleotide, an unlabeled second nucleotide, an unlabeled third nucleotide, an unlabeled fourth nucleotide; or,
when the first nucleotide mixture comprises a first nucleotide labeled with a first label, a second nucleotide labeled with a second label, a third nucleotide labeled with a third label, and an unlabeled fourth nucleotide, or comprises a first nucleotide labeled with a first label, a second nucleotide labeled with a second label, a third nucleotide co-labeled with the first label and the second label and an unlabeled fourth nucleotide, the second nucleotide mixture comprises an unlabeled first nucleotide, an unlabeled second nucleotide and an unlabeled third nucleotide.

In certain embodiments, wherein, the second nucleotide mixture comprises an unlabeled first nucleotide, an unlabeled second nucleotide, an unlabeled third nucleotide, and a first irreversible blocking nucleotide, a second irreversible blocking nucleotide, a third irreversible blocking nucleotide, and a fourth irreversible blocking nucleotide.

In certain embodiments, wherein, the second nucleotide mixture further comprises an unlabeled fourth nucleotide.

In certain embodiments, wherein, the first label, the second label, the third label and the fourth label are each independently the same or different.

In certain embodiments, the first label, the second label, the third label and the fourth label are different.

In certain embodiments, the first label, the second label, the third label and the fourth label are luminescent labels (e.g., fluorescent labels).

In certain embodiments, the first label, the second label, the third label and the fourth label are each independently selected from the group consisting of coumarin, AlexaFluor, Bodipy, fluorescein, tetramethylrhodamine, phenoxazine, acridine, Cy5, Cy3, AF532, Texas Red and derivatives thereof.

In certain embodiments, the target polynucleotide comprises or is DNA, RNA, or any combination thereof. In certain embodiments, the extension product of the nucleic acid molecule is DNA.

In certain embodiments, the target polynucleotide is obtained from a sample derived from of eukaryote (e.g., animal, plant, fungus), prokaryote (e.g., bacterium, actinomycete), virus, phage, or any combination thereof.

In certain embodiments, the first nucleotide, the second nucleotide, the third nucleotide and the fourth nucleotide are each independently selected from the group consisting of A, T, C, G, and U.

In certain embodiments, the first nucleotide, the second nucleotide, the third nucleotide and the fourth nucleotide are each different.

In certain embodiments, the first nucleotide, the second nucleotide, the third nucleotide and the fourth nucleotide are A, T, C, G, respectively.

In certain embodiments, the first irreversible blocking nucleotide, the second irreversible blocking nucleotide, the third irreversible blocking nucleotide and the fourth irreversible blocking nucleotide are each independently selected from A, T, C, G, U.

In certain embodiments, the first irreversible blocking nucleotide, the second irreversible blocking nucleotide, the third irreversible blocking nucleotide and the fourth irreversible blocking nucleotide are different.

In certain embodiments, the first irreversible blocking nucleotide, the second irreversible blocking nucleotide, the third irreversible blocking nucleotide and the fourth irreversible blocking nucleotide are A, T, C, G, respectively.

In certain embodiments, the irreversible blocking nucleotide is a dideoxynucleotide.

In some embodiments, taking photo to detect a light signal is performed while the polymerization reaction in step (d) is performed.

In certain embodiments, the buffer reagent is selected from the group consisting of Trizma base, tris(hydroxymethyl)methylglycine (TRICINE), *N*,*N*-dihydroxyethylglycine (BICINE), tris(hydroxymethyl)methylaminopropane sulfonic acid (TAPS), or any combination thereof.

In certain embodiments, the reagent for nucleic acid hybridization is selected from the group consisting of sodium chloride, potassium chloride, or any combination thereof.

In certain embodiments, the Mg²⁺-containing salt is selected from the group consisting of magnesium sulfate, magnesium chloride, magnesium nitrate, magnesium chromate, or any combination thereof.

In certain embodiments, the surfactant is selected from the group consisting of 10% Tween 20, Tween 80, laureth (brij-35), polyethylene glycol octylphenyl ether (Trinton X-100), ethylphenyl polyethylene glycol (NP-40), sodium dodecyl sulfate (SDS), cetyltrimethylammonium bromide (CTAB), cetyltrimethylammonium chloride (CTAC), or any combination thereof.

In certain embodiments, the reagent for reducing nucleic acid damage under laser photography is selected from the group consisting of sodium ascorbate, dithiothreitol (DTT), 6-hydroxy-2,5,7,8-tetramethylchromane-2-carboxylic acid (Trolox), ascorbic acid derivative (e.g., sodium ascorbyl phosphate, magnesium ascorbyl phosphate, ascorbyl glucoside, ascorbyl palmitate, tetraethyldecanol ascorbate, ascorbyl methylsilanol pectin acid ester, 3-O-ethyl ascorbic acid, reduced glutathione (GSH), *N*,*N'*-dimethylthiourea (DMTU), ammonium pyrrolidine dithiocarbamate (APDTC), spermidine, spermidine trihydrochloride, uric acid, sodium pyruvate, L-cysteine, β-mercaptoethylamine, cystamine, or any combination thereof.

In certain embodiments, the reagent for reducing nucleic acid damage under laser photography is sodium ascorbate or reduced glutathione (GSH).

In one embodiment, the reagent for reducing nucleic acid damage under laser photography is selected from the group consisting of reduced glutathione (GSH) and dithiothreitol (DTT) and any combination thereof.

In one embodiment, the reagent for reducing nucleic acid damage under laser photography is reduced glutathione (GSH).

### Definition of Terms

Unless otherwise defined, all technical and scientific terms used herein have the same meaning as commonly understood by one of ordinary skill in the art to which this invention belongs. All patents, applications, and other publications mentioned herein are incorporated by reference in their entirety. To the extent that definitions set forth herein conflict or are inconsistent with definitions set forth in patents, applications, and other publications incorporated herein by reference, the definitions set forth herein shall prevail.

As used herein, the term "polynucleotide" refers to a deoxyribonucleic acid (DNA), ribonucleic acid (RNA), or the like. Polynucleotides can be single-stranded, double-stranded, or contain both single- and double-stranded sequences. Polynucleotide molecules can be derived from double-stranded DNA (dsDNA) forms (e.g., genomic DNA, PCR and amplification products, etc.), or can be derived from single-stranded forms of DNA (ssDNA) or RNA and can be converted into dsDNA forms, and vice versa. The exact sequence of a polynucleotide molecule may be known or unknown. The followings are illustrative examples of polynucleotides: gene or gene fragment (e.g., probe, primer, EST or SAGE tag), genomic DNA, genomic DNA fragment, exon, intron, messenger RNA (mRNA), transporter RNA, ribosomal RNA, ribozyme, cDNA, recombinant polynucleotide, synthetic polynucleotide, branched polynucleotide, plasmid, vector, isolated DNA of any sequence, isolated RNA of any sequence, and nucleic acid probe, primer or amplification copy of any of the foregoing sequences.

Polynucleotides may include nucleotides or nucleotide analogs. Nucleotides usually contain a saccharide (e.g., ribose or deoxyribose), a base, and at least one phosphate group. Nucleotides can be abasic (i.e., lacking bases). Nucleotides include deoxyribonucleotides, modified deoxyribonucleotides, ribonucleotides, modified ribonucleotides, peptide nucleotides, modified peptide nucleotides, modified sugar-phosphate backbone nucleotides and mixtures thereof. Examples of nucleotides include, for example, adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine triphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP) and deoxyuridine triphosphate (dUTP). Nucleotide analogs containing modified bases may also be used in the methods described herein. Exemplary modified bases that may be contained in polynucleotides, whether with a native backbone or similar structures, include, for example, inosine, xathanine, hypoxathanine, isocytosine, isoguanine, 2-aminopurine, 5-methylcytosine, 5-hydroxymethylcytosine, 2-aminoadenine, 6-methyladenine, 6-methylguanine, 2-propylguanine, 2-propyladenine, 2-thiouracil, 2-thiothymine, 2-thiocytosine, 15-halogenated uracil, 15-halogenated cytosine, 5-propynyluracil, 5-propynylcytosine, 6-azouracil, 6-azocytosine, 6-azothymine, 5-uracil, 4-thiouracil, 8-halogenated adenine or guanine, 8-aminoadenine or guanine, 8-thioadenine or guanine, 8-thioalkyladenine or guanine, 8-hydroxyadenine or guanine, 5-halogenated uracil or cytosine, 7-methylguanine, 7-methyladenine, 8-azaguanine, 8-azaadenine, 7-deazaguanine, 7-deazaadenine, 3-deazaguanine, 3-deazaadenine, etc. As is known in the art, certain nucleotide analogs cannot be incorporated into polynucleotides, for example, nucleotide analogs such as adenosine 5'-phosphoryl sulfate.

In general, nucleotides include nucleotides A, C, G, T or U. As used herein, the term "nucleotide A" refers to a nucleotide containing adenine (A) or a modification or analog thereof, such as ATP, dATP. "Nucleotide G" refers to a nucleotide containing guanine (G) or a modification or analog thereof, such as GTP, dGTP. "Nucleotide C" refers to a nucleotide containing cytosine (C) or a modification or analog thereof, such as CTP, dCTP. "Nucleotide T" refers to a nucleotide containing thymine (T) or a modification or analog thereof, such as TTP, dTTP. "Nucleotide U" refers to a nucleotide containing uracil (U) or a modification or analog thereof, such as UTP, dUTP.

As used herein, the term "dideoxynucleotide" refers to a nucleotide with deoxygenation on the 2'- and 3'-carbons of ribose, also known as 2',3'-dideoxynucleotide. Generally, dideoxynucleotide include dideoxynucleotide A, C, G, T or U. The term "dideoxynucleotide A" refers to a dideoxynucleotide containing adenine (A) or analog thereof, such as ddATP. "Dideoxynucleotide G" refers to a dideoxynucleotide containing guanine (G) or analog thereof, such as ddGTP. "Dideoxynucleotide C" refers to a dideoxynucleotide containing cytosine (C) or analog thereof, such as ddCTP. "Dideoxynucleotide T" refers to a dideoxynucleotide containing thymine (T) or analog thereof, such as ddTP. "Dideoxynucleotide U" refers to a dideoxynucleotide containing uracil (U) or analog thereof, such as ddUTP. As used herein, ddNTP refers to one of dideoxyadenosine triphosphate (ddATP), dideoxyguanosine triphosphate (ddGTP), dideoxycytidine triphosphate (ddCTP), dideoxyuridine triphosphate (ddUTP), dideoxythymidine triphosphate (ddTTP) or a combination of two or more thereof, in which dideoxyuridine triphosphate and dideoxythymidine triphosphate do not appear at the same time.

As used herein, the term "label" refers to a group capable of emitting a luminescent signal under certain conditions.

As used herein, the term "luminescent label" refers to any substance capable of emitting fluorescence at a specific emission wavelength when excited by a suitable excitation wavelength. Such luminescent label may be a chemiluminescent label, for example, selected from biochemiluminescent labels that trigger different luminescence kinetics, and any combination thereof, for example, the chemiluminescent label is selected from luciferases that trigger different luminescence kinetics or any combinaiton thereof; such luminescent label may be, for example, a fluorophore selected from coumarin, AlexaFluor, Bodipy, fluorescein, tetramethylrhodamine, phenoxazine, acridine, Cy5, Cy3, EF700, AF532, Texas Red and derivative thereof, etc.

As used herein, the term "protecting group" refers to a group that prevents a polymerase (which incorporates a nucleotide containing the group into a polynucleotide chain being synthesized) to continuously catalyze the incorporation of another nucleotide after the nucleotide containing the group is incorporated into the polynucleotide chain being synthesized. Such protecting group is also referred to herein as 3'-OH protecting group. Nucleotides containing such protecting group are also referred to herein as 3'-blocked nucleotides. A protecting group can be any suitable group that can be added to a nucleotide as long as the protecting group prevents another nucleotide molecule from being added to the polynucleotide chain, and is easily removed from the sugar portion of the nucleotide without damaging the polynucleotide chain. Furthermore, nucleotides modified with protecting groups need to be resistant to polymerases or other suitable enzymes used to incorporate the modified nucleotides into the polynucleotide chain. Therefore, ideal protecting groups exhibit long-term stability, can be efficiently incorporated by polymerases, prevent secondary incorporation or further incorporation of nucleotides, and can be removed under mild conditions, preferably aqueous conditions, without damaging the polynucleotide structure.

The prior art has described a variety of protecting groups conforming to the above description. For example, WO91/06678 discloses that 3'-OH protecting groups include ester and ether, -F, - NH₂, -OCH₃, -N₃, -OPO₃, -NHCOCH₃, 2-nitrobenzene carbonate, 2,4-sulfenyldinitro and tetrahydrofuran ether. Metzker et al. (Nucleic Acids Research, 22(20):4259-4267, 1994) disclose the synthesis and application of eight 3'-modified 2-deoxyribonucleoside 5'-triphosphates (3'-modified dNTPs). WO2002/029003 describes the use of allyl protecting groups to cap 3'-OH groups on growing DNA chains in the polymerase reaction. Preferably, various protecting groups reported in International Application Publications WO2014139596 and WO2004/018497 may be used, including for example those exemplary protecting groups in Fig. 1A and those 3'-hydroxyl protecting groups (i.e., protecting groups) specified in the claims of WO2014139596, and for example those protecting groups illustrated in Fig. 3 and Fig. 4 and those protecting groups specified in the claims of WO2004/018497. The above references are incorporated by reference in their entirety.

As used herein, the term "reversible blocking group" refers to a group that, when incorporated into a polynucleotide chain being synthesized, renders the polymerase unable to proceed to the next round of polymerization, thereby causing the termination of polymerization reaction, in which case, in each round of polymerization, one and only one base is incorporated into the growing nucleic acid chain; in addition, this group can be removed, and subsequently, the growing nucleic acid chain can undergo the next round of polymerization, and a base is introduced again. Examples of reversible blocking groups are that the H in 3'-OH is substituted with the following groups: ester, ether, -F, -NH₂, -OCH₃, -N₃, -OPO₃, -NHCOCH₃, 2-nitrobenzene carbonate, 2,4-sulfenyldinitro and tetrahydrofuran ether -CH₂-CH=CH₂, S-S, or that the base is blocked with a large steric hindrance group and a fluorescent group and linked to fluorescence via S-S.

As used herein, the term "irreversible blocking nucleotide" refers to a nucleotide that, upon incorporation into a polynucleotide chain being synthesized, prevents the subsequent incorporation of subsequent nucleotides into the polynucleotide chain due to its blocking effect. And this blocking effect is irreversible. The irreversible blocking nucleotides usually include dideoxynucleotides, or nucleotides in which the 3'-OH is replaced by a methoxy group at the 3' end (3'-OMe, or 3'-OCH₃), and nucleotides with 3'-end azide (3'-N₃) and 3'-end ethoxy (3'-OEt, 3'-OCH₂CH₃).

### Beneficial technical effects of the present invention

In sequencing, the present invention provides a technical solution for polymerizing nucleotides while collecting signals to achieve higher polymerization efficiency, in which the solution during signal collection is optimized so that it can perform an additional round of polymerization reaction while collecting signals, the polymerized nucleotides are nucleotides that have no fluorescence modification and only bear blocking groups, preferably nucleotides modified with reversible blocking groups, followed by irreversible blocking nucleotides, or a mixture of the two, and this improves the polymerization efficiency, greatly shortens the overall reaction time of sequencing, and achieves more efficient polymerization efficiency.

### Brief Description of the Drawings

Fig. 1 shows the flow chart of the control group and the experimental group for sequencing *E. coli* DNA by synthesis.
Fig. 2 shows the Q30 (%) proportion results for each cycle of the control group 1 and the experimental group. Among them, the abscissa is the number of sequencing cycles, and the ordinate is the Q30 (%) proportion of each cycle.
Fig. 3 shows the sequencing error rate (%) results for each cycle of the control group 1 and the experimental group. Among them, the abscissa is the number of sequencing cycles, and the ordinate is the sequencing error rate (%) of each cycle.
Fig. 4 shows the lag (%) proportion results for each cycle of the control group 1 and the experimental group. Among them, the abscissa is the number of sequencing cycles, and the ordinate is the lag (%) proportion of each cycle.
Fig. 5 shows the Q30 (%) proportion results for each cycle of the control group 2 and the experimental group 4. Among them, the abscissa is the number of sequencing cycles, and the ordinate is the proportion of Q30 (%) of each cycle.
Fig. 6 shows the sequencing error rate (%) results for each cycle of the control group 2 and the experimental group 4. Among them, the abscissa is the number of sequencing cycles, and the ordinate is the sequencing error rate (%) of each cycle.
Fig. 7 shows the lag (%) proportion results for each cycle of the control group 2 and the experimental group 4. Among them, the abscissa is the number of sequencing cycles, and the ordinate is the lag (%) proportion of each cycle.

### Specific Models for Carrying Out the present invention

The present invention will now be described with reference to the following examples which are intended to illustrate but not to limit the present invention.

Unless otherwise specified, the molecular biology experimental methods used in the present invention basically refer to J. Sambrook et al., Molecular Cloning: Laboratory Manual, 2nd Edition, Cold Spring Harbor Laboratory Press, 1989, and F. M. Ausubel et al., Compiled Experimental Guide to Molecular Biology, 3rd Edition, John Wiley & Sons, Inc., 1995. Those skilled in the art will appreciate that the examples describe the present invention by way of example and are not intended to limit the scope sought to be protected by the present invention.
1. Key equipment used in the examples: MGISEQ-2000RS sequencer, MGIDL-200H loader, MGISEQ-2000RS sequencing slide, MGISEQ-2000RS high-throughput sequencing reagent set, MGISEQ-200RS sequencer, MGISEQ-200RS high-throughput sequencing reagent set, MGISEQ-200RS sequencing slide.
2. The key reagents used in the examples are shown in Table 1 below:

**Table 1. Reagents as used**

| Reagent name | Brand | Item No. |
|---|---|---|
| MGISEQ-2000RS high-throughput sequencing kit | MGI | 1000012552 |
| MGISEQ-200RS high-throughput sequencing kit | MGI | 1000019841 |
| Cold dATP | BGI | 01CATP000-10ml |
| Cold dTTP | BGI | 01CTTP000-10ml |
| Cold dGTP | BGI | 01CGTP000-10ml |
| Cold dCTP | BGI | 01CCTP000-10ml |
| ddATP | BGI | 01DATP000-1ml |
| ddTTP | BGI | 01DTTP000-1ml |
| ddGTP | BGI | 01DGTP000-1ml |
| ddCTP | BGI | 01DCTP000-1ml |

### Example 1

In this example, various reagents were prepared in advance.

### 1) Preparation of Nucleotide Mixture Solution 1

As shown in Table 2 below, all nucleotides in Nucleotide Mixture Solution 1 only had reversible blocking groups, in which A, T, G, and C were adenine nucleotide, thymine nucleotide, guanine nucleotide, and cytosine nucleotide, respectively.

**Table 2: Nucleotide Mixture Solution 1**

| Reagent name | Final concentration (nmol/L) |
|---|---|
| Cold dATP | 200 |
| Cold dTTP | 200 |
| Cold dGTP | 200 |
| Cold dCTP | 200 |

Among them, Cold dATP referred to adenine nucleotide modified only with reversible blocking group, Cold dTTP referred to thymine nucleotide modified only with reversible blocking group, Cold dGTP referred to guanine nucleotide modified only with reversible blocking group, and Cold dCTP referred to cytosine nucleotide only modified with reversible blocking group.

### 2) Preparation of Nucleotide Mixture Solution 2

As shown in Table 3 below, all nucleotides in Nucleotide Mixture Solution 2 are dideoxynucleotides, in which ddATP referred to adenine triphosphate dideoxynucleotide, ddTTP referred to thymine triphosphate dideoxynucleotide, ddGTP referred to guanine triphosphate dideoxynucleotide, ddCTP referred to cytosine triphosphate dideoxynucleotide.

**Table 3: Nucleotide Mixture Solution 2**

| Reagent name | Final concentration (nmol/L) |
|---|---|
| ddATP | 200 |
| ddTTP | 200 |
| ddGTP | 200 |
| ddTTP | 200 |

### 3) Preparation of Nucleotide Mixture Solution 3

As shown in Table 4 below, all nucleotides in Nucleotide Mixture Solution 3 only had reversible blocking groups, in which A, T and C were adenine nucleotide, thymine nucleotide, and cytosine nucleotide, respectively.

**Table 4: Nucleotide Mixture Solution 3**

| Reagent name | Final concentration (nmol/L) |
|---|---|
| Cold dATP | 200 |
| Cold dTTP | 200 |
| Cold dCTP | 200 |

Among them, Cold dATP referred to adenine nucleotide modified only with reversible blocking group, Cold dTTP referred to thymine nucleotide modified only with reversible blocking group, and Cold dCTP referred to cytosine nucleotide modified only with reversible blocking groups.

### 4) Preparation of Photographic Polymerization Solution 1

According to Table 5 below, Photographic Polymerization Solution 1 was prepared and adjusted to pH 8.7 with NaOH and HCl, mixed well for later use.

**Table 5: Photographic Polymerization Solution 1**

| Reagent name | Working concentration |
|---|---|
| Trizma base | 0.05M |
| Sodium chloride | 0.01M |
| Magnesium sulfate | 3mM |
| 10% Tween-20 | 0.05% |
| Reduced glutathione | 0.1M |
| Nucleotide Mixture Solution 1 | 3uM |
| DNA polymerase | 0.01 mg/ml |
| Supplemented with water to final volume 50ml | |

### 5) Preparation of Photographic Polymerization Solution 2

According to Table 6, Photographic Polymerization Solution 2 was prepared and adjusted to pH 8.7 with NaOH and HCl, mixed well for later use.

**Table 6: Photographic Polymerization Solution 2**

| Reagent name | Working concentration |
|---|---|
| Trizma base | 0.05M |
| Sodium chloride | 0.01M |
| Magnesium sulfate | 3mM |
| 10% Tween-20 | 0.05% |
| Reduced glutathione | 0.1M |
| Nucleotide Mixture Solution 2 | 3uM |
| DNA polymerase | 0.01 mg/ml |
| Supplemented with water to final volume 50ml | |

### 6) Preparation of Photographic Polymerization Solution 3

According to Table 7, Photographic Polymerization Solution 3 was prepared and adjusted to pH 8.7 with NaOH and HCl, mixed well for later use.

**Table 7: Photographic Polymerization Solution 3**

| Reagent name | Working concentration |
|---|---|
| Trizma base | 0.05M |
| Sodium chloride | 0.01M |
| Magnesium sulfate | 3mM |
| 10% Tween-20 | 0.05% |
| Reduced glutathione | 0.1M |
| Nucleotide Mixture Solution 1 | 2uM |
| Nucleotide Mixture Solution 2 | 1uM |
| DNA polymerase | 0.01 mg/ml |
| Supplemented with water to final volume 50ml | |

### 7) Preparation of Photography Solution

According to Table 8, Photography Solution was prepared and adjusted to pH 8.2 with NaOH and HCl, mixed well for later use.

**Table 8: Photography Solution**

| Reagent name | Working concentration |
|---|---|
| Trizma base | 0.05M |
| Sodium chloride | 0.01M |
| 10% Tween-20 | 0.05% |
| Reduced glutathione | 0.1M |
| Supplemented with water to final volume 50ml | |

### 8) Preparation of Photographic Polymerization Solution 4

According to Table 9, Photographic Polymerization Solution 4 was prepared and adjusted to pH 8.7 with NaOH and HCl, mixed well for later use.

**Table 9: Photographic Polymerization Solution 4**

| Reagent name | Working concentration |
|---|---|
| Trizma base | 0.05M |
| Sodium chloride | 0.01M |
| Magnesium sulfate | 3mM |
| 10% Tween-20 | 0.05% |
| Reduced glutathione | 0.1M |
| Nucleotide Mixture Solution 3 | 3uM |
| DNA polymerase | 0.01 mg/ml |
| Supplemented with water to final volume 50ml | |

### Example 2

In all the following experiments, *E. coli* single-stranded circular DNA was used as a template, the MGISEQ-2000RS high-throughput sequencing kit was used to complete the preparation of DNA nanospheres that were loaded into the chip for subsequent sequencing.

The nucleotide mixture solution with fluorescence modification and reversible blocking mentioned in this example comprised: dATP-1, which referred to adenine nucleotide with both reversible blocking group modification and Cy5 fluorescence modification; dTTP-1, which referred to thymine nucleotide with both reversible blocking group modification and ROX fluorescence modification; dGTP-1, which referred to guanine nucleotide with both reversible blocking group modification and Cy3 fluorescence modification; and dCTP-1, which referred cytosine nucleotide with both reversible blocking group modification and EF700 fluorescence modification. (With different platforms, the nucleotide mixture solution with fluorescent modification and reversible blocking could be diverse. For example, the nucleotide mixture solution could be incorporated with only nucleotide having reversible blocking group or other types of modification.)

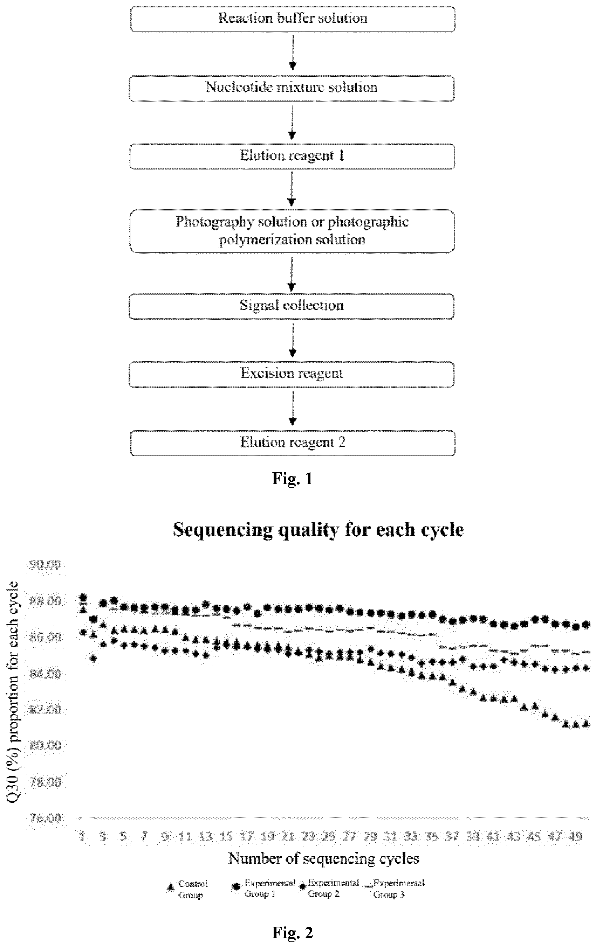

Control Group 1: The MGISEQ-2000RS high-throughput sequencing kit was used, the #10 well reagent in the kit was removed and replaced with the Photographic Polymerization Solution as prepared above. SE50 sequencing was performed on the MGISEQ-2000RS sequencing platform according to the experimental process as shown in Fig. 1. In short, the nucleotide mixture solutions with fluorescence modification and reversible blocking successively underwent polymerization on the MGISEQ-2000RS sequencing platform, and then the free nucleotides were eluted with elution reagent, the signal was collected under the photography solution, the protective groups were removed with excising reagent, and the elution reagent were used to perform washing step. Then, the Q30 decline of each cycle and the sequencing error rate curve of each cycle were calculated to evaluate the sequencing quality.

Experimental Group 1: The MGISEQ-2000RS high-throughput sequencing kit was used, the nucleotide mixture solutions with fluorescence modification and reversible blocking successively underwent polymerization on the MGISEQ-2000RS sequencing platform, the free nucleotides were then eluted with elution reagent, and then the #10 well reagent in the kit was removed and replaced with the Photographic Polymerization Solution 1 of the present invention, so that the signals could be collected at the same time as the polymerization replenishment was performed. At this time, the polymerized nucleotide mixture solution was nucleotides modified with reversibly blocking group, and SE50 sequencing was performed on the MGISEQ-2000RS sequencing platform according to the same experimental process as the control group. Then, the Q30 decline for each cycle and the sequencing error rate curve for each cycle were calculated to evaluate the sequencing quality.

Experimental Group 2: The MGISEQ-2000RS high-throughput sequencing kit was used, the nucleotide mixture solutions with fluorescence modification and reversible blocking successively underwent polymerization on the MGISEQ-2000RS sequencing platform, the free nucleotides were then eluted with elution reagent, and then the #10 well reagent in the kit was removed and replaced with the Photographic Polymerization Solution 2 of the present invention, so that the signals could be collected at the same time as the polymerization replenishment was performed. At this time, the polymerized nucleotide mixture solution was dideoxynucleotides, and SE50 sequencing was performed on the MGISEQ-2000RS sequencing platform according to the same experimental process as the control group. Then, the Q30 decline for each cycle and the sequencing error rate curve for each cycle were calculated to evaluate the sequencing quality.

Experimental Group 3: The MGISEQ-2000RS high-throughput sequencing kit was used, the nucleotide mixture solutions with fluorescence modification and reversible blocking successively underwent polymerization on the MGISEQ-2000RS sequencing platform, the free nucleotides were then eluted with elution reagent, and then the #10 well reagent in the kit was removed and replaced with the Photographic Polymerization Solution 3 of the present invention, so that the signals could be collected at the same time as the polymerization replenishment was performed. At this time, the polymerized nucleotide mixture solution was a mixture solution of reversibly blocking group-modified nucleotides and dideoxynucleotides, and SE50 sequencing was performed on the MGISEQ-2000RS sequencing platform according to the same experimental process as the control group. Then, the Q30 decline for each cycle and the sequencing error rate curve for each cycle were calculated to evaluate the sequencing quality.

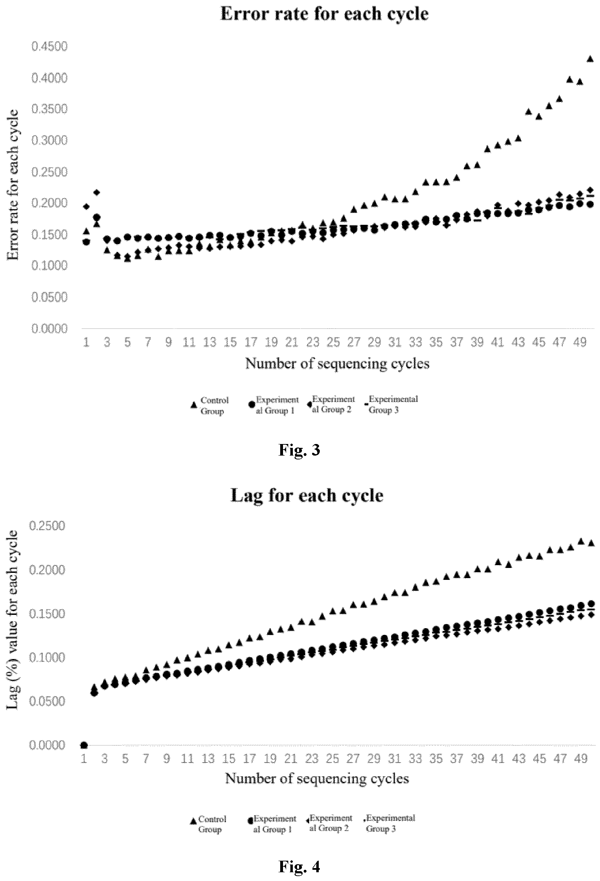

Results: As shown in Fig. 2 to Fig. 4 and Tables 10 to 12 below, Fig. 2 showed the Q30 (%) proportion results for each sequencing cycle corresponding to the above conditions, in which the abscissa was the number of sequencing cycles, and the ordinate was the Q30 (%) proportion for each cycle. Fig. 3 showed the sequencing error rate (%) results under the above conditions, in which the abscissa was the number of sequencing cycles, and the ordinate was the sequencing error rate (%) for each cycle. Fig. 4 showed the lag (%) proportion results for each sequencing cycle corresponding to the above conditions, in which lag is an indicator used to measure whether the sequencing reaction is complete, and refer to the proportion of some copies that are reacted to the N-1 position when sequencing proceed to the N position, the abscissa was the number of sequencing cycles, and the ordinate was the lag (%) proportion for each cycle.

The smaller the Q30 (%) decline for each cycle, the lower the sequencing error rate (%), and the lower the increase in lag (%), indicating better and more stable sequencing quality. As shown in Table 10, the control group showed the greatest Q30 (%) decline after 50 cycles, which was worse than all experimental groups. Experimental Group 1 was slightly better than Experimental Group 3, and Experimental Group 3 was slightly better than Experimental Group 2. As shown in Table 11, the control group showed an error rate that was significantly higher than that of all experimental groups, in which Experimental Group 1 was slightly better than Experimental Group 3, and Experimental Group 3 was slightly better than Experimental Group 2. As shown in Table 12, the control group showed an increase in lag (%) after 50 cycles that was significantly higher than that of all experimental groups, and there was no significant difference between the experimental groups.

**Table 10: Q30 (%) proportion for each cycle**

| Cycle number | Control Group 1 | Experimental Group 1 | Experimental Group 2 | Experimental Group 3 |
|---|---|---|---|---|
| 1 | 87.55 | 88.20 | 86.27 | 87.85 |
| 2 | 86.20 | 87.01 | 84.81 | 87.14 |
| 3 | 86.76 | 87.89 | 85.58 | 87.74 |
| 4 | 86.39 | 88.02 | 85.81 | 87.57 |
| 5 | 86.48 | 87.70 | 85.56 | 87.55 |
| 6 | 86.43 | 87.63 | 85.58 | 87.48 |
| 7 | 86.41 | 87.65 | 85.51 | 87.40 |
| 8 | 86.50 | 87.69 | 85.41 | 87.36 |
| 9 | 86.43 | 87.69 | 85.25 | 87.33 |
| 10 | 86.35 | 87.50 | 85.24 | 87.29 |
| 11 | 86.02 | 87.52 | 85.25 | 87.27 |
| 12 | 85.91 | 87.51 | 85.10 | 87.21 |
| 13 | 85.90 | 87.79 | 85.00 | 87.23 |
| 14 | 85.81 | 87.61 | 85.42 | 87.26 |
| 15 | 85.75 | 87.56 | 85.56 | 87.11 |
| 16 | 85.76 | 87.49 | 85.46 | 86.66 |
| 17 | 85.59 | 87.69 | 85.46 | 86.66 |
| 18 | 85.62 | 87.32 | 85.32 | 86.52 |
| 19 | 85.55 | 87.66 | 85.28 | 86.48 |
| 20 | 85.54 | 87.55 | 85.29 | 86.49 |
| 21 | 85.47 | 87.56 | 85.08 | 86.28 |
| 22 | 85.27 | 87.57 | 85.15 | 86.35 |
| 23 | 85.07 | 87.64 | 85.30 | 86.50 |
| 24 | 84.89 | 87.59 | 85.19 | 86.39 |
| 25 | 85.01 | 87.50 | 85.10 | 86.30 |
| 26 | 84.97 | 87.59 | 85.19 | 86.39 |
| 27 | 84.95 | 87.44 | 85.18 | 86.38 |
| 28 | 84.80 | 87.38 | 85.19 | 86.39 |
| 29 | 84.68 | 87.36 | 85.32 | 86.52 |
| 30 | 84.47 | 87.36 | 85.14 | 86.34 |
| 31 | 84.36 | 87.25 | 85.08 | 86.28 |
| 32 | 84.29 | 87.18 | 85.05 | 86.25 |
| 33 | 84.11 | 87.24 | 84.89 | 86.14 |
| 34 | 83.93 | 87.20 | 84.57 | 86.10 |
| 35 | 83.89 | 87.25 | 84.68 | 86.15 |
| 36 | 83.85 | 86.98 | 84.62 | 85.48 |
| 37 | 83.54 | 86.89 | 84.64 | 85.39 |
| 38 | 83.21 | 86.98 | 84.78 | 85.48 |
| 39 | 83.06 | 87.03 | 84.39 | 85.53 |
| 40 | 82.72 | 87.02 | 84.40 | 85.52 |
| 41 | 82.72 | 86.74 | 84.39 | 85.24 |
| 42 | 82.62 | 86.72 | 84.76 | 85.22 |
| 43 | 82.64 | 86.60 | 84.61 | 85.10 |
| 44 | 82.18 | 86.77 | 84.55 | 85.27 |
| 45 | 82.24 | 87.01 | 84.53 | 85.51 |
| 46 | 81.79 | 87.01 | 84.28 | 85.51 |
| 47 | 81.62 | 86.76 | 84.26 | 85.26 |
| 48 | 81.23 | 86.76 | 84.23 | 85.26 |
| 49 | 81.22 | 86.58 | 84.32 | 85.08 |
| 50 | 81.28 | 86.68 | 84.31 | 85.18 |

**Table 11: Error rate (%) for each cycle**

| Cycle number | Control Group 1 | Experimental Group 1 | Experimental Group 2 | Experimental Group 3 |
|---|---|---|---|---|
| 1 | 0.1560 | 0.1381 | 0.1942 | 0.1401 |
| 2 | 0.1678 | 0.1771 | 0.2170 | 0.1781 |
| 3 | 0.1257 | 0.1431 | 0.1405 | 0.1441 |
| 4 | 0.1166 | 0.1401 | 0.1169 | 0.1400 |
| 5 | 0.1121 | 0.1461 | 0.1152 | 0.1471 |
| 6 | 0.1166 | 0.1441 | 0.1220 | 0.1465 |
| 7 | 0.1267 | 0.1461 | 0.1250 | 0.1451 |
| 8 | 0.1151 | 0.1441 | 0.1272 | 0.1436 |
| 9 | 0.1243 | 0.1451 | 0.1288 | 0.1447 |
| 10 | 0.1247 | 0.1471 | 0.1329 | 0.1468 |
| 11 | 0.1242 | 0.1441 | 0.1307 | 0.1455 |
| 12 | 0.1360 | 0.1461 | 0.1285 | 0.1448 |
| 13 | 0.1313 | 0.1491 | 0.1280 | 0.1451 |
| 14 | 0.1422 | 0.1491 | 0.1303 | 0.1421 |
| 15 | 0.1336 | 0.1451 | 0.1309 | 0.1455 |
| 16 | 0.1400 | 0.1471 | 0.1314 | 0.1503 |
| 17 | 0.1392 | 0.1521 | 0.1330 | 0.1541 |
| 18 | 0.1461 | 0.1481 | 0.1338 | 0.1556 |
| 19 | 0.1532 | 0.1551 | 0.1397 | 0.1569 |
| 20 | 0.1562 | 0.1491 | 0.1415 | 0.1572 |
| 21 | 0.1572 | 0.1551 | 0.1395 | 0.1594 |
| 22 | 0.1664 | 0.1521 | 0.1462 | 0.1615 |
| 23 | 0.1600 | 0.1551 | 0.1472 | 0.1583 |
| 24 | 0.1688 | 0.1531 | 0.1430 | 0.1602 |
| 25 | 0.1699 | 0.1571 | 0.1497 | 0.1624 |
| 26 | 0.1761 | 0.1581 | 0.1517 | 0.1641 |
| 27 | 0.1903 | 0.1591 | 0.1567 | 0.1627 |
| 28 | 0.1967 | 0.1601 | 0.1602 | 0.1644 |
| 29 | 0.1997 | 0.1571 | 0.1632 | 0.1632 |
| 30 | 0.2099 | 0.1631 | 0.1612 | 0.1656 |
| 31 | 0.2068 | 0.1661 | 0.1635 | 0.1649 |
| 32 | 0.2068 | 0.1671 | 0.1621 | 0.1659 |
| 33 | 0.2185 | 0.1671 | 0.1626 | 0.1681 |
| 34 | 0.2339 | 0.1741 | 0.1696 | 0.1726 |
| 35 | 0.2342 | 0.1701 | 0.1753 | 0.1701 |
| 36 | 0.2343 | 0.1741 | 0.1650 | 0.1646 |
| 37 | 0.2416 | 0.1801 | 0.1735 | 0.1799 |
| 38 | 0.2600 | 0.1751 | 0.1817 | 0.1739 |
| 39 | 0.2615 | 0.1831 | 0.1863 | 0.1723 |
| 40 | 0.2871 | 0.1841 | 0.1793 | 0.1859 |
| 41 | 0.2929 | 0.1831 | 0.1967 | 0.1919 |
| 42 | 0.2985 | 0.1831 | 0.1873 | 0.1849 |
| 43 | 0.3038 | 0.1841 | 0.1993 | 0.1865 |
| 44 | 0.3466 | 0.1891 | 0.1967 | 0.1831 |
| 45 | 0.3389 | 0.1891 | 0.2016 | 0.1919 |
| 46 | 0.3557 | 0.1931 | 0.2044 | 0.1959 |
| 47 | 0.3670 | 0.1961 | 0.2135 | 0.2056 |
| 48 | 0.3982 | 0.1941 | 0.2090 | 0.2039 |
| 49 | 0.3948 | 0.1991 | 0.2146 | 0.2079 |
| 50 | 0.4307 | 0.1981 | 0.2209 | 0.2119 |

**Table 12: Lag (%) for each cycle**

| Cycle number | Control Group 1 | Experimental Group 1 | Experimental Group 2 | Experimental Group 3 |
|---|---|---|---|---|
| 1 | 0.0000 | 0.0000 | 0.0000 | 0.0000 |
| 2 | 0.0666 | 0.0594 | 0.0588 | 0.0592 |
| 3 | 0.0720 | 0.0674 | 0.0672 | 0.0676 |
| 4 | 0.0759 | 0.0692 | 0.0686 | 0.0690 |
| 5 | 0.0779 | 0.0711 | 0.0698 | 0.0701 |
| 6 | 0.0791 | 0.0741 | 0.0723 | 0.0731 |
| 7 | 0.0858 | 0.0769 | 0.0748 | 0.0759 |
| 8 | 0.0891 | 0.0786 | 0.0767 | 0.0777 |
| 9 | 0.0922 | 0.0809 | 0.0785 | 0.0799 |
| 10 | 0.0971 | 0.0819 | 0.0796 | 0.0808 |
| 11 | 0.0996 | 0.0846 | 0.0816 | 0.0832 |
| 12 | 0.1036 | 0.0863 | 0.0830 | 0.0847 |
| 13 | 0.1081 | 0.0880 | 0.0853 | 0.0867 |
| 14 | 0.1096 | 0.0899 | 0.0870 | 0.0884 |
| 15 | 0.1142 | 0.0920 | 0.0885 | 0.0903 |
| 16 | 0.1172 | 0.0941 | 0.0903 | 0.0923 |
| 17 | 0.1222 | 0.0962 | 0.0925 | 0.0943 |
| 18 | 0.1235 | 0.0981 | 0.0935 | 0.0959 |
| 19 | 0.1295 | 0.1001 | 0.0953 | 0.0978 |
| 20 | 0.1322 | 0.1021 | 0.0975 | 0.0996 |
| 21 | 0.1343 | 0.1039 | 0.0981 | 0.1020 |
| 22 | 0.1411 | 0.1059 | 0.1003 | 0.1033 |
| 23 | 0.1406 | 0.1079 | 0.1028 | 0.1054 |
| 24 | 0.1472 | 0.1095 | 0.1041 | 0.1069 |
| 25 | 0.1532 | 0.1119 | 0.1058 | 0.1089 |
| 26 | 0.1535 | 0.1139 | 0.1083 | 0.1113 |
| 27 | 0.1605 | 0.1155 | 0.1095 | 0.1126 |
| 28 | 0.1609 | 0.1178 | 0.1117 | 0.1148 |
| 29 | 0.1642 | 0.1197 | 0.1133 | 0.1167 |
| 30 | 0.1694 | 0.1216 | 0.1144 | 0.1190 |
| 31 | 0.1742 | 0.1231 | 0.1166 | 0.1200 |
| 32 | 0.1742 | 0.1256 | 0.1180 | 0.1219 |
| 33 | 0.1802 | 0.1275 | 0.1197 | 0.1237 |
| 34 | 0.1855 | 0.1292 | 0.1216 | 0.1252 |
| 35 | 0.1868 | 0.1318 | 0.1240 | 0.1279 |
| 36 | 0.1923 | 0.1339 | 0.1251 | 0.1294 |
| 37 | 0.1945 | 0.1356 | 0.1265 | 0.1312 |
| 38 | 0.1943 | 0.1374 | 0.1286 | 0.1330 |
| 39 | 0.2009 | 0.1392 | 0.1303 | 0.1349 |
| 40 | 0.2007 | 0.1407 | 0.1314 | 0.1362 |
| 41 | 0.2091 | 0.1431 | 0.1324 | 0.1379 |
| 42 | 0.2061 | 0.1449 | 0.1344 | 0.1398 |
| 43 | 0.2142 | 0.1466 | 0.1360 | 0.1414 |
| 44 | 0.2164 | 0.1490 | 0.1380 | 0.1437 |
| 45 | 0.2157 | 0.1512 | 0.1402 | 0.1458 |
| 46 | 0.2229 | 0.1532 | 0.1419 | 0.1477 |
| 47 | 0.2229 | 0.1553 | 0.1438 | 0.1497 |
| 48 | 0.2259 | 0.1566 | 0.1453 | 0.1520 |
| 49 | 0.2329 | 0.1591 | 0.1471 | 0.1541 |
| 50 | 0.2307 | 0.1612 | 0.1486 | 0.1549 |

### Example 3

In all the following experiments, *E. coli* single-stranded circular DNA was used as template, and MGISEQ-200RS high-throughput sequencing kit was used to complete the preparation of DNA nanospheres that were loaded into the chip for subsequent sequencing.

The nucleotide mixture solutions with fluorescence modification and reversible blocking mentioned in this example comprised: dATP-1, which referred to adenine nucleotide with both reversible blocking group modification and Cy3 and Cy5 fluorescence modification; dTTP-1, which referred to thymine nucleotide with both reversible blocking group modification and Cy5 fluorescence modification; dGTP-1, which referred to guanine nucleotide with only reversible blocking group modification; and dCTP-1, which referred to cytosine nucleotide with both reversible blocking group modification and Cy3 fluorescence modification. (With different platforms, the nucleotide mixture solutions with fluorescent modification and reversible blocking could be diverse. For example, the nucleotide mixture solutions could be incorporated with only reversible blocking group nucleotide or other types of modification.)

Control Group 2: The MGISEQ-200RS high-throughput sequencing kit was used, the #18 well reagent of the kit was removed and replaced with the photography solution prepared above, and SE50 sequencing was carried out on the MGISEQ-200RS sequencing platform according to the experimental process in Fig. 1. Briefly, the nucleotide mixture solutions with fluorescence modification and reversible blocking group successively underwent polymerization on the MGISEQ-200RS sequencing platform, then the free nucleotides were eluted with an elution reagent, the signals were collected under the photography solution, the protecting group was removed by using an excision reagent, and washing was carried out by using an elution reagent. Then, the Q30 decline for each cycle and the sequencing error rate curve for each cycle were calculated to evaluate the sequencing quality.

Experimental Group 4: The MGISEQ-200RS high-throughput sequencing kit was used, the nucleotide mixture solutions with fluorescence modification and reversible blocking successively underwent polymerization on the MGISEQ-200RS sequencing platform (same as Control Group 2), then the free nucleotides were eluted with an elution reagent, then the #18 well reagent of the kit was removed and replaced with the Photographic Polymerization Solution 4 of the present invention, so that the signals could be collected at the same time as the polymerization replenishment was performed. At this time, the polymerized nucleotide mixture solution was reversibly blocking-modified nucleotides and SE50 sequencing was performed on the MGISEQ-200RS sequencing platform according to the same experimental process as Control Group 2. Then, the Q30 decline for each cycle and the sequencing error rate curve for each cycle were calculated to evaluate the sequencing quality.

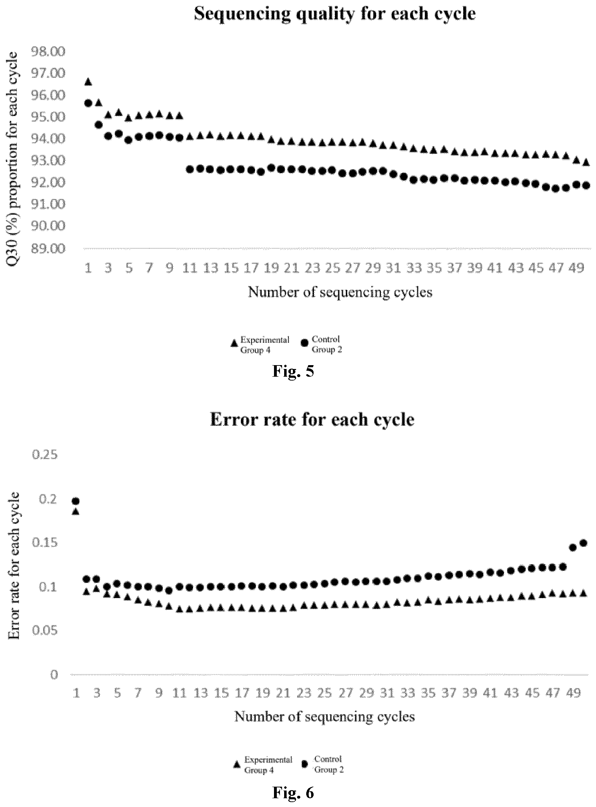

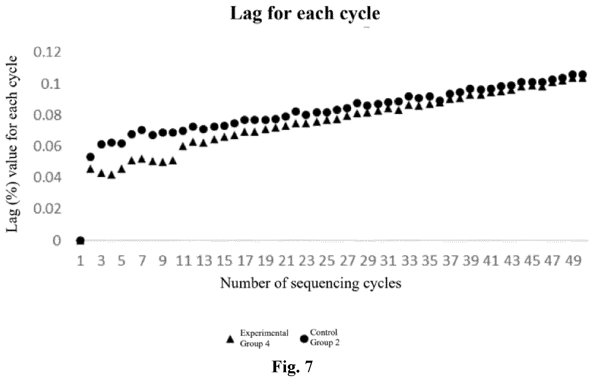

Results: As shown in Fig. 5 to Fig. 7 and Tables 13 to 15 below, Fig. 5 showed the Q30 (%) proportion results for each sequencing cycle corresponding to the above conditions, in which the abscissa was the number of sequencing cycles and the ordinate was the Q30 (%) proportion for each cycle. Fig. 6 showed the sequencing error rate (%) proportion results under the above conditions, in which the abscissa was the number of sequencing cycles, and the ordinate was the sequencing error rate (%) proportion for each cycle. Fig. 7 showed the lag (%) proportion for each sequencing cycle corresponding to the above conditions, in which lag is an indicator used to measure whether the sequencing reaction is complete, which refer to the proportion of copies thereof that merely reacted to the position N-1 when the sequencing proceed to the position N, the abscissa was the number of sequencing cycles, and the ordinate was the lag (%) proportion for each cycle.

The smaller the Q30 (%) decline per cycle, the lower the sequencing error rate (%), and the lower the increase in lag (%), indicating better and more stable sequencing quality. It could be seen from Table 13 that the decrease in Q30 (%) of the control group after 50 cycles was worse than that of Experimental Group 4. As shown in Table 14, the error rate of the control group was significantly higher than that of Experimental Group 4. It could be seen from Table 15 that the increase in lag (%) of the control group after 50 cycles was significantly higher than that of Experimental Group 4.

**Table 13: Q30 (%) proportion for each cycle**

| Cycle number | Experimental Group 4 | Control Group 2 |
|---|---|---|
| 1 | 96.63 | 95.64 |
| 2 | 95.68 | 94.63 |
| 3 | 95.13 | 94.14 |
| 4 | 95.23 | 94.25 |
| 5 | 94.97 | 93.93 |
| 6 | 95.08 | 94.11 |
| 7 | 95.14 | 94.14 |
| 8 | 95.16 | 94.15 |
| 9 | 95.07 | 94.08 |
| 10 | 95.07 | 94.07 |
| 11 | 94.12 | 92.61 |
| 12 | 94.17 | 92.64 |
| 13 | 94.19 | 92.62 |
| 14 | 94.13 | 92.58 |
| 15 | 94.15 | 92.60 |
| 16 | 94.15 | 92.62 |
| 17 | 94.13 | 92.59 |
| 18 | 94.11 | 92.52 |
| 19 | 93.99 | 92.67 |
| 20 | 93.91 | 92.61 |
| 21 | 93.90 | 92.63 |
| 22 | 93.88 | 92.62 |
| 23 | 93.85 | 92.54 |
| 24 | 93.82 | 92.54 |
| 25 | 93.88 | 92.56 |
| 26 | 93.87 | 92.44 |
| 27 | 93.82 | 92.43 |
| 28 | 93.87 | 92.49 |
| 29 | 93.78 | 92.53 |
| 30 | 93.73 | 92.54 |
| 31 | 93.72 | 92.41 |
| 32 | 93.65 | 92.27 |
| 33 | 93.57 | 92.15 |
| 34 | 93.55 | 92.17 |
| 35 | 93.51 | 92.12 |
| 36 | 93.54 | 92.20 |
| 37 | 93.42 | 92.20 |
| 38 | 93.39 | 92.11 |
| 39 | 93.37 | 92.13 |
| 40 | 93.44 | 92.12 |
| 41 | 93.34 | 92.08 |
| 42 | 93.36 | 92.04 |
| 43 | 93.34 | 92.06 |
| 44 | 93.28 | 91.99 |
| 45 | 93.27 | 91.94 |
| 46 | 93.32 | 91.79 |
| 47 | 93.27 | 91.73 |
| 48 | 93.24 | 91.77 |
| 49 | 93.06 | 91.92 |
| 50 | 92.96 | 91.88 |

**Table 14: Error rate (%) for each cycle**

| Cycle number | Experimental Group 4 | Control Group 2 |
|---|---|---|
| 1 | 0.186 | 0.197 |
| 2 | 0.095 | 0.109 |
| 3 | 0.098 | 0.109 |
| 4 | 0.092 | 0.1 |
| 5 | 0.091 | 0.104 |
| 6 | 0.089 | 0.102 |
| 7 | 0.085 | 0.1 |
| 8 | 0.083 | 0.1 |
| 9 | 0.081 | 0.098 |
| 10 | 0.078 | 0.096 |
| 11 | 0.075 | 0.1 |
| 12 | 0.075 | 0.099 |
| 13 | 0.076 | 0.099 |
| 14 | 0.077 | 0.1 |
| 15 | 0.077 | 0.1 |
| 16 | 0.077 | 0.1 |
| 17 | 0.077 | 0.101 |
| 18 | 0.076 | 0.101 |
| 19 | 0.076 | 0.1 |
| 20 | 0.076 | 0.101 |
| 21 | 0.076 | 0.1 |
| 22 | 0.077 | 0.102 |
| 23 | 0.079 | 0.102 |
| 24 | 0.079 | 0.103 |
| 25 | 0.079 | 0.104 |
| 26 | 0.08 | 0.105 |
| 27 | 0.08 | 0.106 |
| 28 | 0.08 | 0.105 |
| 29 | 0.08 | 0.106 |
| 30 | 0.079 | 0.106 |
| 31 | 0.08 | 0.106 |
| 32 | 0.083 | 0.108 |
| 33 | 0.082 | 0.11 |
| 34 | 0.083 | 0.11 |
| 35 | 0.085 | 0.112 |
| 36 | 0.084 | 0.111 |
| 37 | 0.085 | 0.113 |
| 38 | 0.086 | 0.114 |
| 39 | 0.085 | 0.115 |
| 40 | 0.086 | 0.114 |
| 41 | 0.087 | 0.117 |
| 42 | 0.088 | 0.116 |
| 43 | 0.088 | 0.118 |
| 44 | 0.09 | 0.12 |
| 45 | 0.09 | 0.121 |
| 46 | 0.091 | 0.122 |
| 47 | 0.093 | 0.122 |
| 48 | 0.092 | 0.123 |
| 49 | 0.093 | 0.144 |
| 50 | 0.093 | 0.15 |

**Table 15: Lag (%) for each cycle**

| Cycle number | Experimental Group 4 | Control Group 2 |
|---|---|---|
| 1 | 0 | 0 |
| 2 | 0.0456 | 0.0534 |
| 3 | 0.0431 | 0.0615 |
| 4 | 0.042 | 0.0623 |
| 5 | 0.0458 | 0.0616 |
| 6 | 0.0513 | 0.0677 |
| 7 | 0.0522 | 0.0703 |
| 8 | 0.0503 | 0.0671 |
| 9 | 0.05 | 0.0689 |
| 10 | 0.0509 | 0.069 |
| 11 | 0.0603 | 0.0701 |
| 12 | 0.0627 | 0.0726 |
| 13 | 0.0624 | 0.071 |
| 14 | 0.0646 | 0.0728 |
| 15 | 0.0661 | 0.0733 |
| 16 | 0.0674 | 0.0747 |
| 17 | 0.0696 | 0.0768 |
| 18 | 0.0694 | 0.0771 |
| 19 | 0.0708 | 0.0771 |
| 20 | 0.0723 | 0.0775 |
| 21 | 0.0731 | 0.0792 |
| 22 | 0.0746 | 0.0823 |
| 23 | 0.0746 | 0.0799 |
| 24 | 0.0757 | 0.0819 |
| 25 | 0.077 | 0.0818 |
| 26 | 0.0777 | 0.0834 |
| 27 | 0.0796 | 0.0847 |
| 28 | 0.0814 | 0.0875 |
| 29 | 0.0816 | 0.0863 |
| 30 | 0.0828 | 0.0871 |
| 31 | 0.0842 | 0.0881 |
| 32 | 0.0835 | 0.0889 |
| 33 | 0.0864 | 0.0918 |
| 34 | 0.086 | 0.0907 |
| 35 | 0.0871 | 0.092 |
| 36 | 0.088 | 0.0893 |
| 37 | 0.0903 | 0.0936 |
| 38 | 0.091 | 0.0947 |
| 39 | 0.093 | 0.097 |
| 40 | 0.0931 | 0.0963 |
| 41 | 0.0949 | 0.097 |
| 42 | 0.0951 | 0.0982 |
| 43 | 0.0964 | 0.0989 |
| 44 | 0.0986 | 0.1013 |
| 45 | 0.0988 | 0.1009 |
| 46 | 0.0986 | 0.1013 |
| 47 | 0.1012 | 0.1025 |
| 48 | 0.1022 | 0.1037 |
| 49 | 0.1039 | 0.1059 |
| 50 | 0.1037 | 0.1057 |

## Claims

1. A method for analyzing a sequence of target polynucleotide, comprising
(a) providing a target polynucleotide;
(b) contacting the target polynucleotide with a primer under a condition that allows hybridization or annealing, thereby forming a partial duplex comprising the target polynucleotide and the primer used as a growing chain;
(c) contacting the partial duplex with a polymerase and a first nucleotide mixture under a condition that allows the polymerase to perform a nucleotide polymerization reaction, thereby extending the growing chain, wherein the first nucleotide mixture comprises at least one kind of nucleotide labeled with a label,
wherein each nucleotide in the first nucleotide mixture comprises on its ribose or deoxyribose moiety a protecting group capable of blocking nucleic acid chain extension (e.g., a protecting group attached via a 2'- or 3'-oxygen atom);
(d) contacting the product of the previous step with a polymerase and a second nucleotide mixture under a condition that allows the polymerase to perform a nucleotide polymerization reaction, thereby extending the growing chain, wherein the second nucleotide mixture comprises at least one (e.g., one, two, three or four) unlabeled nucleotide or irreversible blocking nucleotide, or a combination of the unlabeled nucleotide and irreversible blocking nucleotide;
wherein each unlabeled nucleotide in the second nucleotide mixture comprises on its ribose or deoxyribose moiety a protecting group capable of blocking nucleic acid chain extension (e.g., a protecting group attached via a 2'- or 3'-oxygen atom);
and, providing a photographic polymerization solution and contacting it with the second nucleotide mixture, and detecting the presence of a label in the product of step (c) through the photographic polymerization reaction;
(e) removing the protective group and label contained in the product of the previous step;
(f) optionally repeating steps (c) to (e) one or more times;
thereby, obtaining a sequence information of the target polynucleotide;
preferably, the photographic polymerization solution comprises the following reagents: a nucleic acid polymerase, a buffer reagent, a surfactant and a reagent for reducing nucleic acid damage under laser photography.

2. The method according to claim 1, wherein in step (c), the extension is an extension using the target polynucleotide as a template; preferably, the extension is an extension of one nucleotide;
preferably, the first nucleotide mixture comprises a first nucleotide labeled with a first label, a second nucleotide labeled with a second label, a third nucleotide labeled with a third label, and a fourth nucleotide labeled with a fourth label or an unlabeled fourth nucleotide, or, a first nucleotide labeled with a first label, a second nucleoside labeled with a second label, a third nucleotide co-labeled with the first label and the second label, and an unlabeled fourth nucleotide.

3. The method according to claim 1 or 2, wherein, in step (d), the second nucleotide mixture comprises an unlabeled first nucleotide, an unlabeled second nucleotide, and an unlabeled third nucleotide;
optionally, the second nucleotide mixture further comprises an unlabeled fourth nucleotide;
optionally, the second nucleotide mixture comprises at least one irreversible blocking nucleotide.

4. The method according to claim 1 or 2, wherein, in step (d), the second nucleotide mixture comprises an unlabeled first nucleotide, an unlabeled second nucleotide, an unlabeled third nucleotide, as well as a first irreversible blocking nucleotide, a second irreversible blocking nucleotide, a third irreversible blocking nucleotide, and a fourth irreversible blocking nucleotide;
optionally, the second nucleotide mixture further comprises an unlabeled fourth nucleotide.

5. The method according to any one of claims 1 to 4, wherein the first label, the second label, the third label and the fourth label are each independently the same or different;
preferably, the first label, the second label, the third label and the fourth label are different; preferably, the first label, the second label, the third label and the fourth label are luminescent labels (e.g., fluorescent labels);
more preferably, the first label, the second label, the third label and the fourth label are each independently selected from the group consisting of coumarin, AlexaFluor, Bodipy, fluorescein, tetramethylrhodamine, phenoxazine, acridine, Cy5, Cy3, AF532, Texas Red and derivative thereof;
preferably, the target polynucleotide comprises or is DNA, RNA, or any combination thereof; preferably, the extension product of the nucleic acid molecule is DNA;
preferably, the target polynucleotide is obtained from a sample derived from eukaryote (e.g., animal, plant, fungus), prokaryote (e.g., bacterium, actinomycete), virus, phage, or any combination thereof;
preferably, the first nucleotide, the second nucleotide, the third nucleotide and the fourth nucleotide are each independently selected from the group consisting of A, T, C, G, and U;
preferably, the first nucleotide, the second nucleotide, the third nucleotide and the fourth nucleotide are different;
further preferably, the first nucleotide, the second nucleotide, the third nucleotide and the fourth nucleotide are A, T, C, G, respectively;
preferably, the first irreversible blocking nucleotide, the second irreversible blocking nucleotide, the third irreversible blocking nucleotide and the fourth irreversible blocking nucleotide are each independently selected from the group consisting of A, T, C, G, and U;
preferably, the first irreversible blocking nucleotide, the second irreversible blocking nucleotide, the third irreversible blocking nucleotide and the fourth irreversible blocking nucleotide are different;
further preferably, the first irreversible blocking nucleotide, the second irreversible blocking nucleotide, the third irreversible blocking nucleotide and the fourth irreversible blocking nucleotide are A, T, C, G, respectively;
preferably, the irreversible blocking nucleotides are dideoxynucleotides;
preferably,
when the first nucleotide mixture comprises a first nucleotide labeled with a first label, a second nucleotide labeled with a second label, a third nucleotide labeled with a third label, and a fourth nucleotide labeled with a fourth label, the second nucleotide mixture comprises an unlabeled first nucleotide, an unlabeled second nucleotide, an unlabeled third nucleotide, and an unlabeled fourth nucleotide; or,
when the first nucleotide mixture comprises a first nucleotide labeled with a first label, a second nucleotide labeled with a second label, a third nucleotide labeled with a third label, and an unlabeled fourth nucleotide, or comprises a first nucleotide labeled with a first label, a second nucleotide labeled with a second label, a third nucleotide co-labeled with the first label and the second label, and an unlabeled fourth nucleotide, the second nucleotide mixture comprises an unlabeled first nucleotide, an unlabeled second nucleotide, and an unlabeled third nucleoside.

6. A kit, which comprises:
(a) a first nucleotide mixture, in which the first nucleotide mixture comprises at least one kind of nucleotide labeled with a label,
wherein each nucleotide in the first nucleotide mixture comprises on its ribose or deoxyribose moiety a protecting group capable of blocking nucleic acid chain extension (e.g., a protecting group attached via a 2'- or 3'-oxygen atom);
(b) a second nucleotide mixture, in which the second nucleotide mixture comprises at least one (e.g., one, two, three, or four) unlabeled nucleotide, or irreversible blocking nucleotide, or a combination of the unlabeled nucleotide and irreversible blocking nucleotide;
wherein each unlabeled nucleotide in the second nucleotide mixture comprises on its ribose or deoxyribose moiety a protecting group capable of blocking nucleic acid chain extension (e.g., a protecting group attached via a 2'- or 3'-oxygen atom);
(c) a photographic polymerization solution;
preferably, the photographic polymerization solution comprises the following reagents: a nucleic acid polymerase, a buffer reagent, a surfactant, a reagent for reducing nucleic acid damage under laser photography;
preferably, the kit further comprises one or more selected from the group consisting of: a primer complementary to all or part of the polynucleotide, a nucleic acid amplification buffer, a working buffer for enzyme (e.g., nucleic acid polymerase), water, or any combination thereof;
preferably, the kit further comprises one or more selected from the group consisting of: a sequencing slide, a reagent for removing the protecting group and label on the nucleotide;
preferably, the kit is used for analyzing a polynucleotide;
preferably, the kit is used for sequencing a polynucleotide.

7. The kit according to claim 6, wherein the first nucleotide mixture comprises a first nucleotide labeled with a first label, a second nucleotide labeled with a second label, a third nucleotide labeled with a third label, and a fourth nucleotide labeled with a fourth label or an unlabeled fourth nucleotide, or comprises a first nucleotide labeled with a first label, a second nucleotide labeled with a second label, a third nucleotide co-labeled with the first label and the second label, and an unlabeled fourth nucleotide.

8. The kit according to claim 6 or 7, when the first nucleotide mixture comprises a first nucleotide labeled with a first label, a second nucleotide labeled with a second label, a third nucleotide labeled with a third label, and a fourth nucleotide labeled with a fourth label, the second nucleotide mixture comprises an unlabeled first nucleotide, an unlabeled second nucleotide, an unlabeled third nucleotide, and an unlabeled fourth nucleotide; or,
when the first nucleotide mixture comprises a first nucleotide labeled with a first label, a second nucleotide labeled with a second label, a third nucleotide labeled with a third label, and an unlabeled fourth nucleotide, or comprises a first nucleotide labeled with a first label, a second nucleotide labeled with a second label, a third nucleotide co-labeled with the first label and the second label, and an unlabeled fourth nucleotide, the second nucleotide mixture comprises an unlabeled first nucleotide, an unlabeled second nucleotide, and an unlabeled third nucleotide.

9. The kit according to any one of claims 6 to 8, wherein the second nucleotide mixture comprises an unlabeled first nucleotide, an unlabeled second nucleotide, an unlabeled third nucleotide, as well as a first irreversible blocking nucleotide, a second irreversible blocking nucleotide, a third irreversible blocking nucleotide and a fourth irreversible blocking nucleotide;
optionally, the second nucleotide mixture further comprises an unlabeled fourth nucleotide.

10. The kit according to any one of claims 6 to 9, wherein the first label, the second label, the third label and the fourth label are each independently the same or different;
preferably, the first label, the second label, the third label and the fourth label are different;
preferably, the first label, the second label, the third label and the fourth label are luminescent labels (e.g., fluorescent labels);
more preferably, the first label, the second label, the third label and the fourth label are each independently selected from the group consisting of coumarin, AlexaFluor, Bodipy, fluorescein, tetramethylrhodamine, phenoxazine, acridine, Cy5, Cy3, AF532, Texas Red and derivative thereof;
preferably, the target polynucleotide comprises or is DNA, RNA, or any combination thereof; preferably, the extension product of the nucleic acid molecule is DNA;
preferably, the target polynucleotide is obtained from a sample derived from eukaryote (e.g., animal, plant, fungus), prokaryote (e.g., bacterium, actinomycete), virus, phage, or any combination thereof;
preferably, the first nucleotide, the second nucleotide, the third nucleotide and the fourth nucleotide are each independently selected from the group consisting of A, T, C, G, and U;
preferably, the first nucleotide, the second nucleotide, the third nucleotide and the fourth nucleotide are different;
further preferably, the first nucleotide, the second nucleotide, the third nucleotide and the fourth nucleotide are A, T, C, and G, respectively;
preferably, the first irreversible blocking nucleotide, the second irreversible blocking nucleotide, the third irreversible blocking nucleotide and the fourth irreversible blocking nucleotide are each independently selected from the group consisting of A, T, C, G, U;
preferably, the first irreversible blocking nucleotide, the second irreversible blocking nucleotide, the third irreversible blocking nucleotide and the fourth irreversible blocking nucleotide are different;
further preferably, the first irreversible blocking nucleotide, the second irreversible blocking nucleotide, the third irreversible blocking nucleotide and the fourth irreversible blocking nucleotide are A, T, C, and G, respectively;
preferably, the irreversible blocking nucleotide is a dideoxynucleotide.
